# EUROPEAN PATENT SPECIFICATION

(11) **EP 0 977 739 B1**
(45) Date of publication and mention of the grant of the patent: **29.10.2003**
(21) Application number: 98910084.7
(22) Date of filing: 23.03.1998
(51) Int. Cl.: C07D 221/16, C07D 213/81, C07C 25/02, C07C 17/16

(54) **SYNTHESIS OF INTERMEDIATES USEFUL IN PREPARING TRICYCLIC COMPOUNDS**
SYNTHESE VON ZWISCHENPRODUKTEN ANWENDBAR IN DER HERSTELLUNG VON TRICYCLISCHEN VERBINDUNGEN
SYNTHESE D'INTERMEDIAIRES UTILES DANS LA PREPARATION DE COMPOSES TRICYCLIQUES

(30) Priority: 25.03.1997 US 823928
(43) Date of publication of application: 09.02.2000
(73) Proprietor: SCHERING CORPORATION, Kenilworth New Jersey 07033 (US)
(72) Inventor: CHEN, Xing, Plainsboro, NJ 08536 (US); POIRIER, Marc, Parlin, NJ 08859 (US); WONG, Yee-Shing, Florham Park, NJ 07932 (US); WU, Guang-Zhong, Neshanic Station, NJ 08853 (US)
(74) Representative: von Kreisler, Alek, Dipl.-Chem.
(86) International application number: US9803812
(87) International publication number: WO98042676

(56) References cited:
- EP-A- 0 011 858
- EP-A- 0 396 083
- WO-A-96/31478
- GB-A- 2 094 790
- KAREL SINDELAR ET AL: "Tricyclic psychotropic agents containing two chalcogen atoms in the central ring : derivatives of 11H-dibenz[b,f]-1,4-oxathiepin" COLLECTION OF CZECHOSLOVAK CHEMICAL COMMUNICATIONS., vol. 47, no. 3, 1982, PRAGUE CS, pages 967-983, XP002069467
- PELZ K ET AL: "NEUROTROPE UND PSYCHOTROPE SUBSTANZEN XXII WEITERE CHLORDERIVATE DES 10-(4-METHYLPIPERAZINO)-10,11-DIHYDRODIBEN ZO[ b,f]THIEPINS UND EINIGE VERWANDTE STOFFE" COLLECTION OF CZECHOSLOVAK CHEMICAL COMMUNICATIONS, vol. 33, no. 6, 1 January 1968, pages 1852-1872, XP000560349

## Description

### BACKGROUND OF THE INVENTION

This invention provides an improved process for preparing intermediates useful in the preparation of tricyclic compounds known as antihistamines and as inhibitors of farnesyl protein transferase (FPT). In particular, the compounds of this invention are useful in the preparation of antihistamines such as those disclosed in U.S. Patents 4,282,233 and 5,151,423, and of FPT inhibitors disclosed in International Application No. PCT/US96/19603, filed December 19, 1996.

### SUMMARY OF THE INVENTION

This invention provides a process for preparing a compound of the formula 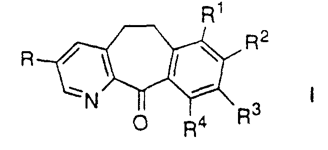 wherein:
R, R¹, R², R³ and R⁴ are independently selected from the group consisting of hydrogen and halo;
comprising:
(a) reacting a compound of formula 1 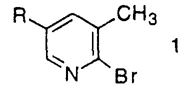
   (i) with an amine of the formula NHR⁵R⁶, wherein R⁵ is hydrogen and R⁶ is C₁-C₆ alkyl, aryl or heteroaryl; R⁵ is C₁-C₆ alkyl, aryl or heteroaryl and R⁶ is hydrogen; R⁵ and R⁶ are independently selected from the group consisting of C₁-C₆ alkyl and aryl; or R⁵ and R⁶, together with the nitrogen to which they are attached, form a ring comprising 4 to 6 carbon atoms or comprising 3 to 5 carbon atoms and one hetero moiety selected from the group consisting of -O- and -NR⁹-, wherein R⁹ is H, C₁-C₆ alkyl or phenyl; in the presence of a palladium catalyst and carbon monoxide to obtain an amide of formula 2: 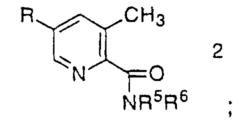 or
   (ii) with an alcohol of the formula R¹⁰OH, wherein R¹⁰ is C₁-C₆ lower alkyl or C₃-C₆ cycloalkyl, in the presence of a palladium catalyst and carbon monoxide to obtain the ester of formula 2A 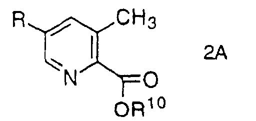 followed by reacting the compound of 2A with an amine of formula NHR⁵R⁶ to obtain the amide of formula 2;
(b) reacting the amide of formula 2 with an iodo-substituted compound of formula 3 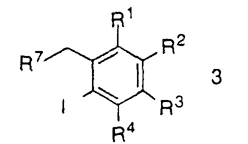 wherein R¹, R², R³ and R⁴ are as defined above and R⁷ is Cl or Br, in the presence of a strong base to obtain a compound of formula 4 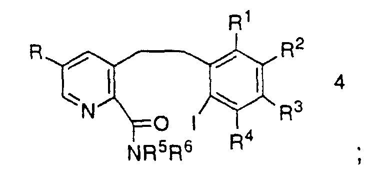
(c) cyclizing a compound of formula 4 with a reagent of the formula R⁸MgL, or when none of R, R¹, R², R³ and R⁴ are bromo, with a reagent of the formula R⁸Li, wherein R⁸ is C₁-C₈ alkyl, aryl or heteroaryl and L is Br or Cl; provided that prior to cyclization, compounds wherein R⁵ or R⁶ is hydrogen are reacted with a suitable N-protecting group.

This invention also claims the intermediate compound of formula 3 wherein R¹ and R³ are hydrogen, R² is chloro, and each of R⁴ and R⁷ are bromo, i.e., a compound of formula 5:

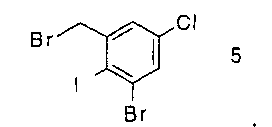

This invention also claims the intermediate compound of formula 4, in particular a compound of formula 4 wherein R¹ and R³ are hydrogen, R² is chloro, and each of R and R⁴ are bromo, i.e., a compound of formula 4A, or wherein R¹, R², R³ and R⁴ are hydrogen and R² is chloro, i.e., a compound of formula 4B:

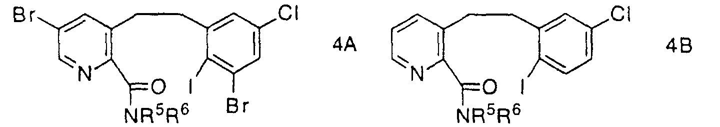

Also claimed herein is a process for preparing a compound of formula 5 comprising:
i) brominating 2-amino chlorobenzoic acid of formula 6 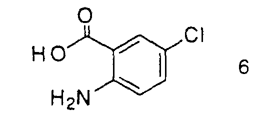 to obtain 2-amino-3-bromo-5-chlorobenzoic acid of formula 7 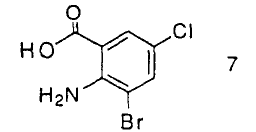
ii) iodonating the compound of formula 7 to obtain 2-iodo-3-bromo-5-chlorobenzoic acid of formula 8 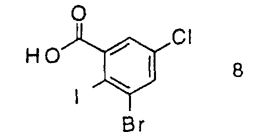
iii) reducing the carboxylic acid of the halo-substituted benzoic acid of formula 8 to obtain the corresponding hydroxy-methyl compound of formula 9 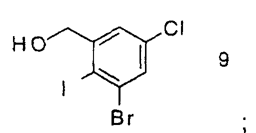 and
iv) brominating the compound of formula 9. A process for preparing a compound of formula 5A 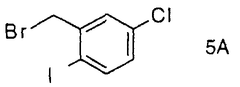 comprises:
   i) iodonating the compound of formula 7A 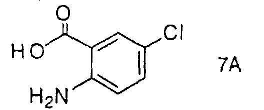 to obtain 2-iodo-5-chlorobenzoic acid of formula 8A 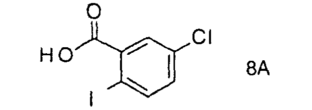
   ii) reducing the carboxylic acid of the halo-substituted benzoic acid of formula 8A to obtain the corresponding hydroxy-methyl compound of formula 9A 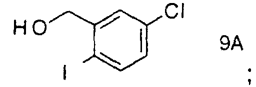 and
   iii) brominating the compound of formula 9A.

Preferred compounds of formula I are those wherein R² is halo. Also preferred are compounds wherein R¹ and R³ are each hydrogen. Another group of preferred compounds is that wherein R, R¹, R³ and R⁴ are hydrogen and R² is halo. Still another group of preferred compounds is that wherein R¹ and R³ are each hydrogen and R and R² are independently selected from the group consisting of halo. Yet another group of preferred compounds is that wherein R¹ and R³ are each hydrogen and R, R² and R⁴ are independently selected from the group consisting of halo. Halo is preferably Cl or Br.

### DETAILED DESCRIPTION

As used herein, the terms "alkyl" and "lower alkyl," where not otherwise defined, mean straight or branched alkyl chains of 1 to 6 carbon atoms.

"Halo" refers to fluorine, chlorine, bromine or iodine radicals.

"Aryl" means phenyl, substituted phenyl wherein the substituents are 1 to 3 substituents independently selected from the group consisting of C₁ to C₆ alkyl and C₁ to C₆ alkoxy, benzyloxy or naphthyl.

"Heteroaryl" means a 5- or 6-membered aromatic ring comprising one or two nitrogen atoms, e.g., pyridyl, pyrimidyl, imidazolyl or pyrrolyl.

When R⁵ and R⁶, together with the nitrogen to which they are attached, form a ring comprising 4 to 6 carbon atoms, the rings so produced are exemplified by pyrrolidinyl, piperidinyl and perhydroazepine. When R⁵ and R⁶, together with the nitrogen to which they are attached, form a ring comprising 4 to 5 carbon atoms and a heteroatom, the rings so produced are exemplified by piperazinyl, N-methyl-piperazinyl, N-phenyl-piperazinyl and morpholinyl.

The compounds prepared by the process disclosed above are useful as intermediates in the procedures described in PCT/US96/19603 and U.S. 5,151,423 to obtain the desired compounds wherein the piperidinyl ring is N-substituted. Using those procedures, the compounds of the present invention are reacted with a substituted piperidine of the formula 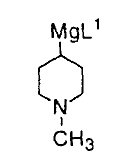 wherein L¹ is a leaving group selected from the group consisting of Cl and Br, to obtain a compound of the formula

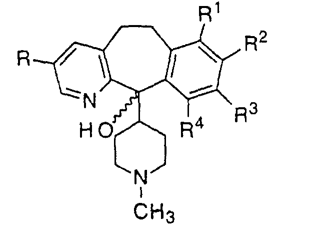

This compound is converted to the corresponding piperidylidene, the nitrogen is deprotected, and the compound is reduced to the piperidyl form. The piperidinyl nitrogen can then be reacted with a variety of compounds, e.g., an acyl compound such as an ester or acyl chloride to form the desired amide.

By using the intermediates prepared by the process of this invention, the desired tricyclic antihistamines and FPT inhibitors described above can be made by a seven-step process rather than the fifteen-step process disclosed in the art. The present process allows halo substitution at any of R¹, R², R³ and/or R⁴, while previously disclosed procedures were not operative for preparing compounds wherein R⁴ is halogen. Moreover, the present process, employing the iodo-substituted intermediate of formula 3, is regioselective, producing the compound of formula 4 in high yield; without the iodo substituent, the reaction of step (b) produces undesirable mixtures of products, for example compounds wherein two compounds of formula 2 react in the presence of the strong base to produce a compound wherein the methyl group of one molecule joins to the carbonyl group of the other.

In step (a), the bromo-substituted pyridine of formula 1 is reacted with the amine NHR⁵R⁶ or with the alcohol of formula R¹⁰OH in the presence of a palladium catalyst, carbon monoxide (CO) and a base; when reacted with the alcohol, the product is then converted to an amide by reaction with an amine of the formula NHR⁵R⁶.

As defined above, the amines of formula NHR⁵R⁶ are exemplified by aniline, N-methylaniline, pyrrolidine, piperidine, perhydroazepine, piperazine, N-methyl-piperazine, N-phenyl-piperazine and morpholine. Preferred amines are aniline and N-methylaniline, with aniline being most preferred. The amount of amine (NHR⁵R⁶) reacted ranges from 1 to 4 equivalents, and is preferably 1 to 1.5 equivalents.

Palladium catalysts are exemplified by PdX₂/ligand at ratios of 1:0.5 to 1:3, preferably 1:1 to 1:2, at a range of 0.5 to 40 mol%, preferably 1 to 10 mol%, and most preferably 1 to 5 mol%; Pd(PPh₃)₄; (R¹¹)₃P/ Pd₂(dba)₃; and Pd/C, wherein X is OAc or Cl, ligand refers to P(R¹¹)₃ or a nitrogen-based ligand such as dipyridyl, 2-aminopyridine, 2-cyanopyridine, 2-dimethylaminopyridine, 1,10-phenanthroline, 2-methoxypyridine or (S)-(-)-nicotine, and wherein Ac is acetyl, R¹¹ is C₁ to C₆ alkyl or aryl, Ph is phenyl, and dba is dibenzylidene acetone. Preferred catalysts are Pd(OAc)₂/dipyridyl, Pd(OAc)₂/P(R¹¹)₃ and (PPh₃)₂PdCl₂.

Suitable bases include, but are not limited to, C₁ to C₁₀ alkyl amines such as triethylamine (Et₃N), t-butylamine and 1,8-diazabicyclo-[5,4,0]undec-7-ene (DBU), and inorganic bases such as K₂CO₃, Na₂CO₃, Na₂HPO₄ and NaOH. Preferred bases are K₂CO₃, DBU and Et₃N, with 1,8-DBU being preferred for use with Pd(OAc)₂/dipyridyl and Et₃N being preferred for use with (PPh₃)₂PdCl₂.

Suitable solvents are tetrahydrofuran (THF), dimethyl-formamide (DMF), acetonitrile (CH₃CN) and toluene or a combination thereof. CH₃CN is preferred for reaction with an amine and a combination of CH₃CN and toluene is preferred for reaction with an alcohol. The temperature range for the reaction is 35°C to 100°C, preferably about 55°C for reaction with the amine and preferably about 80°C for reaction with an alcohol. The reaction is carried out at a pressure of 5 psi to 500 psi, preferably 40 to 200 psi, and most preferably at 50 to 150 psi. The time for reaction ranges from 2 hours to 4 days, preferably 4 hours to 2 days, and most preferably 16 to 48 hours.

Conversion of the ester of formula 2A to the amide of formula 2 is accomplished by methods well known in the art, for example by reacting the ester directly with the amine or by using the conditions described by Basha et al in Tetrahedron Letters, (1977), p. 4171.

In step (b), the amide formed in step (a) is reacted with the iodo-substituted compound of formula 3 in a solvent such as THF, t-butyl methyl ether (t-BuOMe), diethyl ether (Et₂O), diglyme or a mixture thereof, preferably a mixture of THF and t-butyl methyl ether, in the presence of a strong base such as lithium diisopropylamide (LDA), lithium hexamethyldisilylamide or soium amide, preferably LDA. The concentration of the base ranges from 2.0 to 4.0 equivalents, preferably 2.0 to 2.2 equivalents. The iodo compound of formula 3 is reacted in a concentration range of 1.0 to 1.5 equivalents, preferably 1.1 equivalents. The reaction is carried out in a temperature range of -78°C to -20°C, preferably -50°C to -30°C.

In step (c), the product of step (b) is cyclized by treating with 1.0 to 3.0 equivalents, preferably 1.1 equivalents, of a reagent of the formula R⁸MgL, wherein R⁸ is C₁-C₆ alkyl such as iso-propyl; aryl such as phenyl, 2,4,6-trimethylphenyl, 2-methylphenyl, 2-methoxy-phenyl, 2-methoxy-5-methylphenyl or 2,5-dimethoxyphenyl; or heteroaryl such as N-methyl-piperidyl and L is Br or Cl. Typical reagents of the formula R⁸MgL are isopropylmagnesium chloride, 2-mesitylmagnesium bromide, o-tolyl-magnesium bromide, 2-methoxy-phenylmagnesium bromide, 2-methoxy-5-methylphenylmagnesium bromide, 2,5-di-methoxyphenyl-magnesium bromide and N-methyl-piperidylmagnesium bromide. A preferred reagent of formula R⁸MgL is one wherein R⁸ is 2-methoxy-phenyl, e.g., 2-methoxyphenylmagnesium bromide. For compounds wherein none of R, R¹, R², R³ and R⁴ are bromo, the cyclization reagent can also be R⁸Li, wherein R⁸ is as defined above. Preferred R⁸Li reagents are n-, sec- and tert-butyllithium, methyllithium and phenyllithium. Suitable solvents include t-BuOMe, Et₂O, THF and toluene, with THF being preferred. The temperature range for the reaction is -78°C to 25°C, preferably 0 to -40°C, and most preferably -25 to -15°C.

Before cyclization, a protecting step is necessary when one of R⁵ or R⁶ is hydrogen. A protecting group can be added either after step (a) or after step (b). A compound of formula 2 or 4 is suitably protected by methods well known in the art, using protecting groups well known in the art, for example by reacting with CH₃l and a base such as NaNH₂, LDA, butyl lithium, NaH, CaH₂ or NaOH with a phase transfer cataylst, preferably NaH or NaOH with a phase transfer cataylst. Suitable phase transfer catalysts include C₁ to C₈ tertiary alkyl amine salts such as tetrabutylammonium bromide, tetrabutylammonium chloride or tetraoctylammonium bromide, benzyltriethylammonium chloride, trialkylsulfates, phosphorus salts and crown ethers. Base concentration ranges from 1 to 3 equivalents, preferably 1.5 equivalents, and phase transfer catalyst concentration ranges from 1.0 to 50 mol%, preferably 10 mol%. CH₃I concentration ranges from 1.0 to 5 equivalents, preferably 1.5 equivalents. Suitable solvents for the methylation step are THF, DMF, N,N-dimethyl-acetamide and dimethylsulfoxide (DMSO), with DMF being preferred. The temperature range for the reaction is -20°C to 20°C, preferably -10°C.

In the process for preparing the intermediate of formula 5, step (i) comprises brominating an amino,halo-substituted benzoic acid of formula 6 to obtain the corresponding 3-bromo-substituted benzoic acid of formula 7 by treating the compound of formula 6 with 1.0 to 2.0 equivalents, preferably 1.5 equivalent of bromine and an acid such as acetic acid (HOAc), HCl, CF₃CO₂H, CH₃SO₃H or CF₃SO₃H, preferably HOAc. The reaction is carried out at a temperature of 0 to 40°C, preferably 10 to 20°C.

In step (ii), the brominated benzoic acid of formula 7 is iodinated to obtain a compound of formula 8 by reacting with NaNO₂ or KNO₂, preferably NaNO₂, in an acid such as HCl, H₂SO₄, CH₃SO₃H or CF₃CO₂H, preferably H₂SO₄, and then treating the resultant product with Kl, Nal or tetrabutylammonium iodide, preferably Kl, in water. The nitrite concentration ranges from 1.0 to 4.0 equivalents, preferably 2.2 equivalents, and the iodide concentration ranges from 2 to 10 equivalents, preferably 5 to 7 equivalents. The reaction temperature ranges from -10 to 40°C, with a preferred range of -5 to 5°C.

In step (iii), the chloro-bromo-iodo benzoic acid of formula 8 is reduced to the corresponding alcohol by methods well known in the art. Suitable reducing agents include, but are not limited to, BH₃•THF and B(OCH₃)₃, BH₃•(CH₃)₂S (BMS) and B(OCH₃)₃, NaBH₄/SOCl₂, KBH₄/SOCl₂, NaBH₄/AlCl₃ and NaBH₄/TiCl₄. Preferred reagents are BH₃•THF or BMS in combination with B(OCH₃)₃ or NaBH₄ in combination with SOCl₂. As an example, the concentration of BMS ranges from 1.0 to 4.0 equivalents, preferaby 2.5 to 3.0 equivalents, and the concentration of B(OCH₃)₃ ranges from 5 to 20 equivalents, preferably 10 to 16 equivalents. The temperature range for the reaction is from 0 to 30°C, preferably 15 to 25°C.

In step (iv) of the process for preparing the intermediate, the hydroxysubstituted compound is converted to the corresponding bromo-substituted compound by treatment with a brominating reagent such as SOBr₂, PPh₃ and Br₂, or Br₃P, preferably PPh₃ and Br₂. The amount of PPh₃ and Br₂ ranges from 1.0 to 2.0 equivalents, preferably being 1.1 to 1.4 equivalents. Suitable solvents are THF, CH₃CN, EtCN and CH₂Cl₂, with CH₃CN being preferred. The temperature range for the reaction is 0 to 20°C, preferably 3 to 8°C.

In the process for preparing the intermediate of formula 5A, the benzoic acid of formula 7A is iodinated to obtain a compound of formula 8A, reduced to the alcohol of formula 9A, and brominated in the same manner as that described for the preparation of the compound of formula 5.

Starting materials of formula 1, NHR⁵R⁶, R⁸MgL and R⁸Li are known in the art or can readily be prepared by one skilled in the art. Starting materials of formula 3 are known in the art or, where the starting material is of formula 5 or 5A, can be prepared by methods disclosed herein.

Following are specific examples of the procedures in the various steps of the process of this invention for preparing compounds of formula I and formula 3, although those skilled in the art will appreciate that similar procedures within the scope of the process of this invention can be used to prepare other compounds of formula I and formula 3.

### Preparation 1

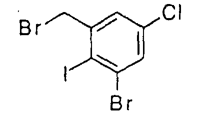

### Step (i):

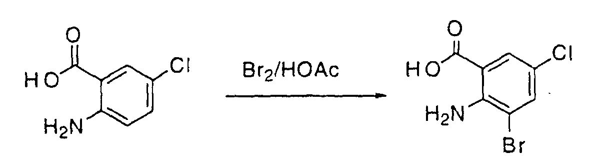

To a solution of 200 g (1.05 mol) of 2-amino-5-chlorobenzoic acid in 3.4 L of HOAc at 15°C was added dropwise 184 g (1.15 mol) of Br₂. The mixture was stirred at 15°C for 4 hrs, quenched slowly into 8 L of water, and extracted with 2 X 2 L of t-BuOMe. The combined extract was washed with water, dried over MgSO₄ and concentrated. The crude product was treated with hot hexane, filtered and dried to give 210 g (80%) of 2-amino-3-bromo-5-chlorobenzoic acid as white solid. Mp. 225-228°C. ¹H NMR (DMSO-d₆): δ 7.70 (d, J = 2.6 Hz, 1 H), 7.69 (d, J = 2.6 Hz, 1 H), 6.8 (bs, 2 H). ¹³C NMR (DMSO-d₆): δ 168.16, 147.06, 136.25, 130.19, 118.22, 112.35, 110.34. IR: 3480 (m), 3350 (m), 2920 (s), 1670 (s) cm⁻¹. Analysis calcd. for C₇H₅BrClNO₂: C, 33.53, H, 2.00, N, 5.59; Found: C, 33.63, H, 2.12, N, 5.70.

### Step (ii):

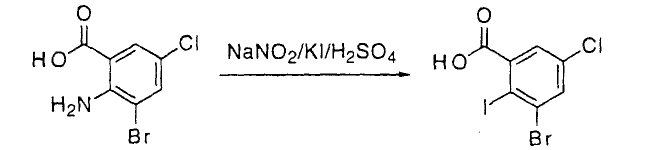

To 40 g (159 mmol) of the product of step (i) in 160 mL of conc. H₂SO₄ at 0°C 24.1 g (350 mmol) of NaNO₂ was added slowly. The mixture was mechanically stirred at that temperature for 3 hrs and quenched into 1 L ice with strong agitation. The resulting solution was added slowly into 158 g (954 mmol) of KI in 2 L ice water and extracted with 2 X 1 L of EtOAc. The combined extract was washed with NaHSO₃, dried over MgSO₄ and concentrated. To the residue was added hexane and the precipitate was filtered and dried to give 50.4 g (87%) of 2-lodo-3-bromo-5-chlorobenzoic acid as white solid. Mp. 174-176°C. ¹H NMR (DMSO-d₆): δ 7.98 (d, J = 2.4 Hz, 1 H), 7.60 (d, J = 2.4 Hz, 1 H). ¹³C NMR (DMSO-d6): δ 168.08, 144.23, 134.13, 133.08, 132.53, 126.91, 99.88. IR: 3150 (m), 2920 (s), 1720 (s), 1650 (m) cm⁻¹.

### Step (iii):

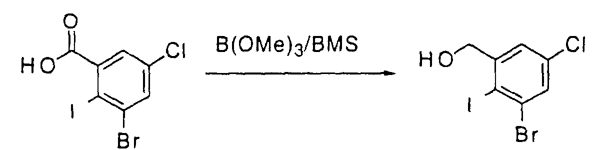

To a 2 L flask with a mechanical stirrer, a thermometer, and an addition funnel, at r.t. were added sequentially 50 g (138 mmol) of the product of step (ii), 500 mL of THF, 229 mL (2.01 mol) of 2.0 M (CH₃O)₃B and 193 mL (386.4 mmol) of 2.0 M BH₃•(CH₃)₂S. The reaction mixture was stirred at r.t. for 18 hrs, quenched with 500 mL of CH₃OH and concentrated. The residue was dissolved with 1 L EtOAc, washed with brine, dried over MgSO₄, and concentrated to give 47 g (98%) of 2-iodo-3-bromo-5-chlorobenzyl alcohol as a white solid.

Alternatively, the acid was reduced following a two-step, one-pot procedure: first, the acid was converted to its corresponding acid chloride and then treated with NaBH₄. Mp. 99-101°C. ¹H NMR (CDCl₃): δ 7.55 (d, J = 2.4 Hz, 1 H), 7.39 (d, J = 2.4 Hz, 1 H), 4.61 (s, 2 H), 2.48 (bs, 1 H). ¹³C NMR (CDCl₃): δ 146.70, 134.93, 130.84, 130.48, 125.74, 100.45, 69.99. IR: 3200 (s), 2920 (s) cm⁻¹. Anal. calcd. for C₇H₇ClBrlO: C, 24.03, H, 2.00; Found: C, 24.35, H, 2.19.

### Step (iv):

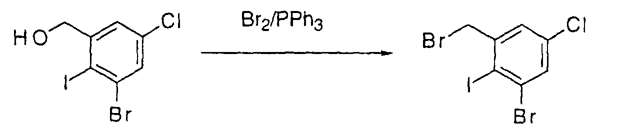

To a 500 mL flask with a mechanical stirrer at 5°C were added 9.7 g (37 mmol) of PPh₃, 100 mL CH₃CN and 6 g (37 mmol) of Br₂. The reaction mixture was stirred at 5°C for 1 hr, and 10 g (28.7 mmol) of the alcohol of step (iii) in 100 mL of CH₃CN was added dropwise. The reaction mixture was allowed to warm to r.t., agitated for 1 hr, and concentrated. The residue was extracted with 2 X 400 mL CH₂Cl₂, washed with brine, dried over MgSO₄ and concentrated. The phosphoxide was filtered after addition of hexane. The filtrate was passed through a pad of silca gel and concentrated to give 11.3 g (96%) of 2-iodo-3-bromo-5-chlorobenzylbromide as a white solid.

Alternatively, the alcohol was converted either to the bromide using SOBr₂ in 90% yield, or to the corresponding chloride using SOCl₂. Mp. 75-77°C. ¹H NMR (CDCl₃): δ 7.54 (d, J = 2,4 Hz, 1 H), 7.36 (d, J = 2.4 Hz, 1 H), 4.60 (s, 2 H). ¹³C NMR (CDCl₃): δ 143.94, 134.66, 131.98, 131.58, 128.15, 104.71, 39.52. IR: 2920 (s), 1540 (m) cm⁻¹.

### Preparation 2

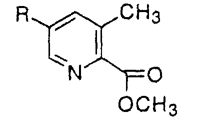

To a 400 mL autoclave was charged 1.6 g (6.06 mmol) of 2,5-dibromo-3-methylpyridine, 0.45 g (0.64 mmol) of (PPh₃)₂PdCl₂, 30 mL of toluene/CH₃CN (1:1), 0.33 mL (95 mmol) of Et₃N and 4 eq. of CH₃OH. The autoclave was sealed, evacuated, flushed with nitrogen three times and charged with carbon monoxide to 80 psi. The autoclave was heated to 80°C for 16 hrs, cooled to r.t. and the excess carbon monoxide was evacuated under vacuum. The conversion was about 55% as determined by NMR. The contents of the autoclave were transferred into a flask for concentration. The residue was then purified on a silica gel column, eluting with hexane:EtOAc to give the ester as a white solid. M.p. 61-62°C. ¹H NMR (CDCl₃): δ 8.58 (d, J = 1.9 Hz, 1H), 7.78 (d, J = 1.9 Hz, 1H), 3.96 (s, 3H), 2.58 (s, 3H). ¹³C NMR (CDCl₃): δ 165.82, 147.94, 145.17, 142.20, 137.63, 123.56, 52,74, 19.96. IR: 1715 cm⁻¹.

### Preparation 3

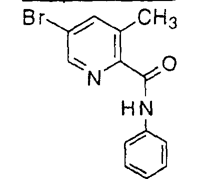

To a 4 L autoclave were added sequentially 250 g (949 mmol) of 2,5-dibromo-3-methylpyridine, 6.7 g (30 mmol) of Pd(OAc)₂, 5.0 g ( 32 mmol) of dipyridyl, 10 L of toluene, 127 mL (1.1 mol) of aniline, and 277 mL (2.0 mol) of DBU. The autoclave was sealed, evacuated, purged with nitrogen, and charged with carbon monoxide to 80 psi. The reaction mixture was heated to 65°C for about 2 days with periodical refilling as necessary, and then cooled to r.t. The contents of the autoclave was vented under vacuum and flushed with nitrogen, then transferred to a 10 L flask with the aid of water and EtOAc. The mixture was concentrated and filtered through a pad of celite. The filtrate was extracted with 2 X 1 L of toluene. The combined extract was washed with brine, filtered and concentrated. The residue was recrystallized from hot *i*-PrOH and the precipitate was filtered, washed with M.L., and dried at 50°C to give 220 g (76%) of the amide as white solid.

### Preparation 4

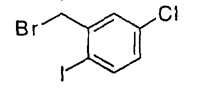

### Step (i):

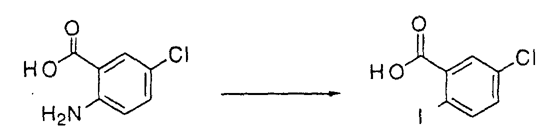

To 75 g (394 mmol) of 2-amino-5-chlorobenzoic acid (90%) in 300 mL of conc. H₂SO₄ at 0°C was added slowly 60 g (870 mmol) of NaNO₂. The mixture was mechanically stirred at that temperature for 5 hrs and at rt for 12 hrs, then quenched into 2 L ice with strong agitation. The resulting solution was added slowly into 393 g (2.37 mol) of Kl in 2 L ice water and extracted with 2 X 1 L of EtOAc. The combined extract was washed with NaHSO₃, dried over MgSO₄, concentrated and dried to give 124 g (>100%) of 2-iodo-5-chlorobenzoic acid as white solid. ¹H NMR (DMSO-d₆): δ 7.91 (d, J = 8.5 Hz, 1H), 7.66 (d, J = 2.6 Hz, 1H), 7.26 (dd, J = 8.5, 2.6, 1H).

### Step (ii):

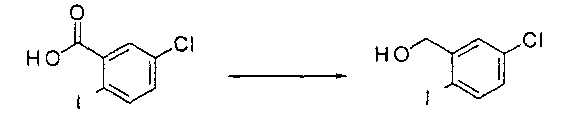

To a 2 L flask with a mechanical stirrer, a thermometer, and an addition funnel at r.t. were added sequentially 124 g (0.4 mol) of the product of Step (i), 700 mL of THF, 500 g (4.85 mol) of (CH₃O)₃B, and 560 mL (1.12 mol) of 2.0 M BH₃•Me₂S. The reaction mixture was stirred at r.t. for 18 hrs., quenched with 500 mL of CH₃OH and concentrated. The residue was dissolved with 1 L EtOAc, washed with brine, dried over MgSO₄, and concentrated to give 121 g (>100%) of 2-iodo-5-chlorobenzyl alcohol as white solid. ¹H NMR (CDCl₃): δ 7.64 (d, J = 8.3 Hz, 1H), 7.41 (d, J = 2.5 Hz, 1H), 6.93 (dd, J = 8.3, 2.5, 1H), 4.57 (s, 2H).

### Step (iii):

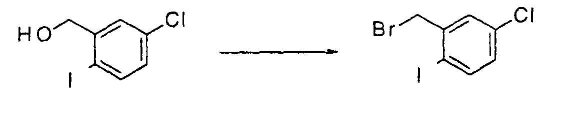

To a 500 mL flask with a mechanical stirrer at 5°C were added 140 g (0.53 mol) of PPh₃, 1100 mL CH₃CN, and 85 g (0.53 mol) of Br₂. The reaction mixture was stirred at 5°C for 1 hr, and 121 g (about 0.4 mmol) of the alcohol of Step (ii) was added portionwise. The reaction mixture was allowed to warm to r.t., agitated for 1 hr, and concentrated. The residue was extracted with 2 X 400 mL CH₂Cl₂, washed with brine, dried over MgSO₄, and concentrated. The phosphoxide was filtered after addition of hexane. The filtrate was passed through a pad of silica gel and concentrated to give 2-iodo-5-chiorobenzylbromide as white solid (about 95% yield). ¹H NMR (CDCl₃): δ 7.68 (d, J = 8.5 Hz, 1H), 7.38 (d, J = 2.5 Hz, 1H), 6.90 (dd, J = 8.5, 2.5 Hz, 1H), 4.44 (s, 2H).

Alternatively, the alcohol was converted either to the bromide using SOBr₂ in 90% yield, or to the corresponding chloride using SOCl₂.

### Example 1

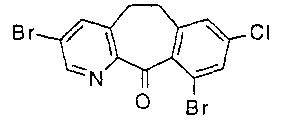

### Step 1:

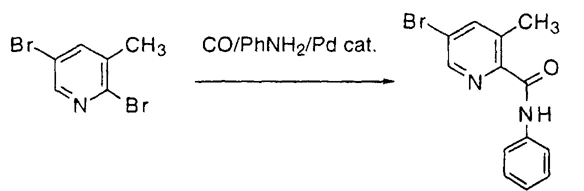

To a 4 L autoclave were added sequentially 250 g (949 mmol) of 2,5-dibromo-3-methylpyridine, 21 g (30 mmol) of (Ph₃P)₂PdCl₂, 2 L of CH₃CN, 100 mL (1.1 mol) of aniline, and 154 mL (1.5 mol) of Et₃N. The autoclave was sealed, evacuated, purged with nitrogen and charged with carbon monoxide to 80 psi. The reaction mixture was heated to 60°C for about 3 days with periodical refilling as necessary, and then cooled to r.t. The contents of the autoclave was vented under vacuum, flushed with nitrogen and transferred to a 10 L flask with the aid of water and EtOAc. The mixtue was concentrated and filtered through a pad of celite. The filtrate was extracted with 2 X 1 L of EtOAc. The comibined extract was washed with brine, dried over MgSO₄, filtered and concentrated. The residue was recrystallized from hot i-PrOH and the precipitate was filtered, washed with mother liquor and dried at 50°C to give 162 g (59%) of the amide as white solid. The solution yield was determined to be 71%. Mp. 103-104°C. ¹H NMR (CDCl₃): δ 10.00 (bs, 1H), 8.49 (d, J = 2.1 Hz, 1H), 7.79 (d, J = 2.1 Hz, 1 H), 7.72 (d, J = 7.5 Hz, 2H), 7.37 (dd, J= 7.5, 7.4 Hz, 2 H), 7.13 (t, J = 7.4 Hz, 1 H), 2.79 (s, 3 H). ¹³C NMR (CDCl₃): δ 162.79, 146.34, 145.21, 143.29, 137.91, 137.72, 128.96, 124.18, 123.12, 119.61, 20.68. IR: 3320 (w), 2920 (s), 1700 (m) cm⁻¹. Elemental analysis: calcd for C₁₃H₁₁BrN₂O: C, 53.60, H, 3.78, N, 9.62; found: C, 53.50, H, 3.79, N, 9.51. Step 2:

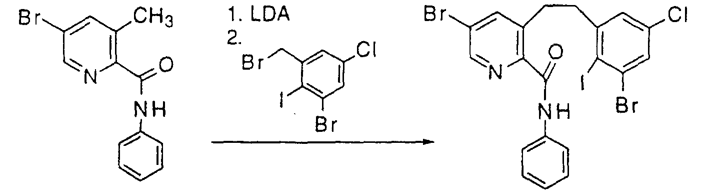

To a solution of 0.98 mL (7 mmol) of i-Pr₂NH in 5 mL t-BuOMe at -40°C was added 2.8 mL (7 mmol) of 2.5 M n-BuLi in hexanes. To a mixture of 1 g (3.44 mmol) of the product of Step 1 in 4 mL of THF and 10 mL of t-BuOMe at -50°C was added dropwise the LDA solution. The resulting purple solution was added dropwise at -40°C into 1.4 g (3.61 mmol) of the product of Preparation 1 in 10 mL of t-BuOMe. The mixture was allowed to warm to 0°C and was quenched with a solution of NH₄Cl. The precipitate was filtered, washed with brine and hexanes, and dried to give 1.47 g (69%) of the desired product. An analytical sample was purified on a silica gel column. Mp. 186-187°C. ¹H NMR (CDCl₃): δ 10.02 (s, 1H), 8.54 (d, J = 2.1 Hz, 1H), 7.78 (d, J = 2.1 Hz, 1H), 7.72 (d, J = 8.4 Hz, 2H), 7.49 (d, J = 2.4 Hz, 1H), 7.39 (dd, J = 7.4, 8.4 Hz, 2H), 7.29 (d, J = 2.4 Hz, 1H), 7.14 (t, J = 7.4 Hz, 1H), 3.44-3.40 (m, 2H), 3.28-3.18 (m, 2H). ¹³C NMR (CDCl₃): δ 162.40, 148.14, 147.14, 145.20, 142.84, 140.16, 137.62, 135.04, 131.48, 130.33, 129.10, 128.14, 124.44, 123.43, 119.83, 105.42, 44.21, 33.67. IR: 2920 (s), 1680 (m) cm⁻¹. HRMS: Calc'd for C₂₀H₁₅Br₂ClIN₂O: 620.8265, Found: 620.8262 (MH⁺).

### Step 3:

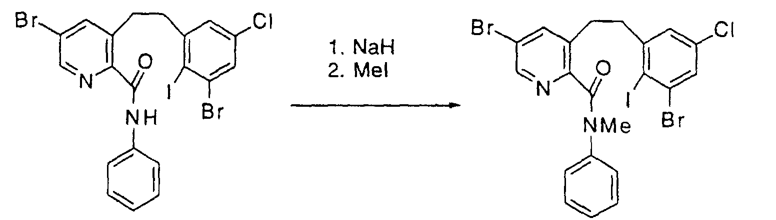

To 27.7 g (44.2 mmol) of the product of Step 2 in 750 mL of DMF at -10°C was added 1.5 g 80% NaH (66.3 mmol). After stirring at -10°C for 1 hr, 4.1 mL (66.3 mmol) of CH₃l was added to the flask. The mixture was mechanically stirred at -10°C for 1 hr, then quenched carefully into 2 L ice. The precipitate was filtered, washed with water and dried to give 23.7 g (85%) of the desired product as an off-white solid. Mp. 180-181°C. NMR indicates two rotamers. ¹H NMR (CDCl₃): δ 8.26 (d, J = 1.8 Hz, 1H), 7.53 (d, J = 1.8 Hz, 1H), 7.52 (s, 1H), 7.18-7.10 (m, 6H), 3.15 (s, 3H), 3.17-3.10 (m, 2H), 2.83-2.79 (m, 2H). ¹³C NMR (CDCl₃): δ 167.61, 152.35, 147.94, 147.59, 142.87, 139.41, 135.11, 131.79, 130.51, 129.06, 129.04, 127.98, 127.04, 126.65, 120.21, 105.08, 43.97, 37.26, 31.96. IR: 2920 (s), 1650 (m) cm⁻¹. HRMS: Calc'd for C₂₁H₁₇Br₂ClIN₂O: 634.8420, Found: 634.8423 (MH⁺).

### Step 4:

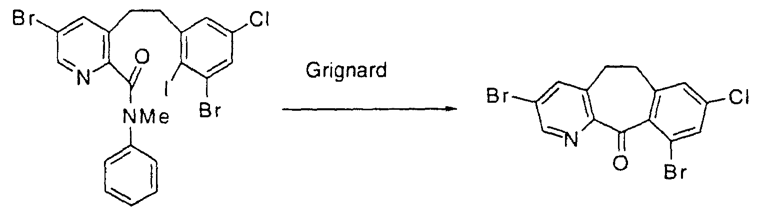

To a solution of 2 g (3.15 mmol) of the product of Step 3 in 40 mL of THF at -20°C was added dropwise 4.8 mL of 0.72 M 2-CH₃OC₆H₄MgBr (3.5 mmol) in THF. The mixture was stirred at -20°C for 20 min. and quenched with 5 mL of saturated NH₄Cl. The quenched solution was stirred at r.t. for 16 hrs. to complete the hydrolysis, concentrated and extracted with 2 X 10 mL of EtOAc. The combined extract was washed with brine, dried over MgSO₄ and concentrated. The residue was chromatographed on silica gel, eluting with hexane/EtOAc (9:1) to give 0.84 g (66%) of the azoketone. The solution yield was determined to be 82% by HPLC. Mp. 198-200°C. ¹H NMR (CDCl₃): δ 8.74 (d, J = 1.8 Hz, 1 H), 7.75 (d, J = 1.8 Hz, 1 H), 7.55 (d, J = 1.8 Hz, 1 H), 7.20 (d, J = 1.8 Hz, 1 H), 3.25-3.19 (m, 2 H), 3.15-3.09 (m, 2 H). ¹³C NMR (CDCl₃): δ 194.17, 150.21. 149.39, 140.90, 140.73, 139.18, 137.69, 136.54, 131.48, 126.79, 123.33, 119.88, 32.78, 31.59. IR: 2920 (s), 1690 (m) cm⁻¹. Elemental analysis: Calcd. for C₁₄H₈Br₂Cl NO: C, 41.84, H, 1.99, N, 3.49; Found: C, 42.11 H, 2.07, N, 3.64.

### Example 1A

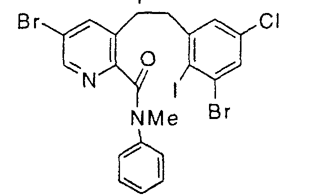

Alternative route to the product of Example 1, Step 3:

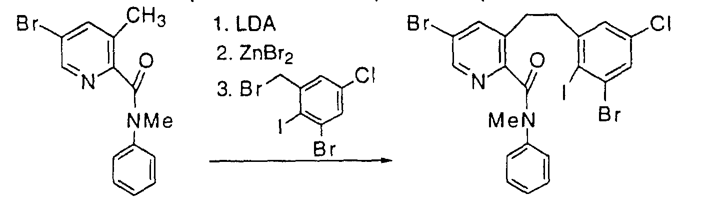

To a solution of 2.5 mL (18 mmol) of diisopropylamine in 10 mL dry THF at -60°C was added dropwise 7.2 mL (18 mmol) of 2.5 M *n*-BuLi. To another flask containing 5 g (16.4 mmol) of the N-methyl amide starting material (prepared in a manner similar to that described in Example 1, Step 1) in 50 mL THF at -78°C was added the above LDA solution. After stirring at -78°C for 5 min., 20.8 mL (18 mmol) of a freshly prepared solution of ZnBr₂ was added. To the resulting mixture was added 6.7 g (16.4 mmol) of 5-chloro-3-bromo-2-2iodobenzyl bromide in 10 mL THF. The reaction was heated to reflux for 2 hrs, quenched slowly into saturated NH₄Cl and extracted with toluene. The combined extract was washed with brine, dried over MgSO₄ and concentrated. The precipitate was filtered to give 4.6 g (44%) of the N-methylated product. The HPLC solution yield was determined to be 60%.

### Example 3

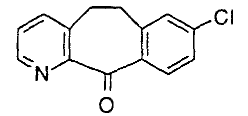

### Step 1:

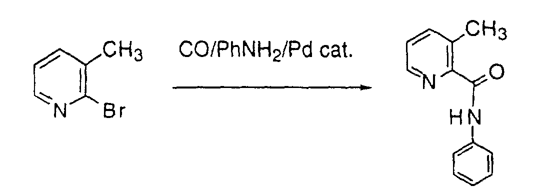

In a 150 mL autoclave were added sequentially 10 g (55 mmol) of 2-bromo-3-methylpyridine, 1.2 g (1.7 mmol) of (Ph₃P)₂PdCl₂, 50 mL of CH₃CN, 8 mL (87 mmol) of aniline, and 18 mL (116 mmol) of DBU. The autoclave was sealed, evacuated, purged with nitrogen and charged with carbon monoxide to 80 psi. The reaction mixture was heated to 65°C for 9 h with periodical refilling of carbon monoxide as necessary, and then cooled to r.t. The contents of the autoclave was vented under vacuum, flushed with nitrogen and transferred into a separatory funnel with the aid of water and EtOAc. The phases were separated and the aqueous phase was extracted with 100 mL of EtOAc. The combined extract was washed with brine, dried over MgSO₄, filtered and concentrated. The residue was recrystallized from hot *i*-PrOH and water and the precipitated was filtered and dried at 50°C to give 6.9 g (59%) of the amide as white solid. The solution yield was determined to be 76%. Mp. 66-67°C. ¹H NMR (CDCl₃): δ 10.23 (bs, 1H), 8.37 (dd, J = 4.6 Hz, 0.8 Hz, 1H), 7.71 (m, 2H), 7.62 (dd, J = 6.95 Hz, 1H), 7.31-7.36 (m, 3H), 7.10 (t, J = 7.42 Hz, 1H), 2.79 (s, 3H). ¹³C NMR (CDCl₃): δ 163.52, 146.70, 145.21, 141.28, 138.02, 136.13, 128.94, 125.95, 123.97, 119.62, 20.80. R: 3330 (w), 2920 (s), 1680 (m) cm⁻¹. Analysis. Calcd for C₁₃H₁₂N₂O: C, 73.58, H, 5.66, N, 13.21; found: C, 73.29, H, 5.76, N, 12.81.

### Step 2

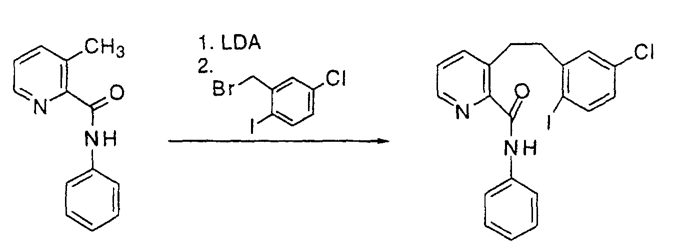

To a solution of 10 mL (70 mmol) of *i*-Pr₂NH in 40 mL *t*-BuOCH₃ at -40°C was added 28 mL (70 mmol) of 2.5 M n-BuLi in hexanes. To a mixture of 7.0 g (33.0 mmol) of the product of Step 1 in 30 mL of THF and 70 mL of *t*-BuOCH₃ at -30°C was added dropwise the above LDA solution. The resulting purple solution was added dropwise at -30°C into 11.0 g (33.0 mmol) of 3-chloro-6-iodo benzyl bromide in 20 mL of THF and 50 mL of *t*-BuOCH₃. The mixture was allowed to warm to 0°C and quenched with a solution NH₄Cl. The phases were separated and the aqueous phase was extracted with 100 mL of *t*-BuOCH₃. The combined organic solution was washed with brine, dried over MgSO₄, filtered and concentrated. The crude product was used directly in the following step without further purification. ¹H NMR (CDCl₃): δ 10.24 (s, 1 H), 8.43 (dd, J = 4.57 Hz, J = 1.6 Hz, 1H), 7.71 (m, 2H), 7.52 (dd, J = 7.8 Hz, 1,59 Hz, 1H), 7.29-7.35 (m, 2H), 7.24 (d, J =2.61 Hz, 1H), 7.08 (t, J = 7.43 Hz, 1H), 6.81 (dd, J = 8.4 Hz, J = 2.6 Hz, 1H), 3.40-3.44 (m, 2H), 3.03-3.07 (m, 2H).

### Step 3:

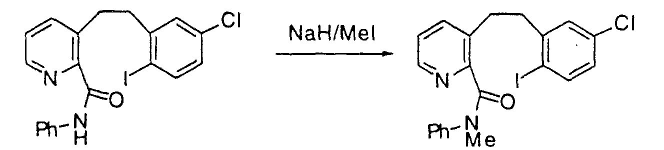

To the residue (∼33 mmol) of the product of Step 2 in 70 mL of DMF at 0°C was added 2.6 g 60% NaH (66 mmol). After stirring at 0°C for 1 hr, 2.5 mL (40 mmol) of CH₃l was added to the flask. The mixture was mechanically stirred at 0°C for 15 min, then quenched carefully by ice. EtOAc (200 mL) was added and the solution was washed with water (100 x 5). The organic layer was concentrated to give 16 g residue, which was separated by column chromatography (hexane/EtOAc) to give 10 g product, 64% yield, in two steps. Mp. 106-107°C. NMR indicate two rotamers. ¹H NMR (CDCl₃): δ 8.17 (d, J = 4.6 Hz, 1H), 7.67 (d, J = 8.5 Hz, 1H), 7.33 (d, J = 7.6 Hz, 1H), 7.13 (d, J = 2.4 Hz, 1H), 6.96 - 7.1 (m, 6H), 6.86 (dd, J = 8.5, 2.4 Hz, 1H), 3.49 (s, 3H), 2.90 - 2.96 (m, 2H), 2.74 -2.80 (m, 2H). ¹³C NMR (CDCl₃): δ 168.4, 153.9, 146.5, 145.4, 143.1, 140.4, 137.0, 134.6, 133.2, 129.7, 128.8, 128.3, 126.7, 126.6, 123.3, 97.2, 41.4, 37.1, 32.1.

### Step 4:

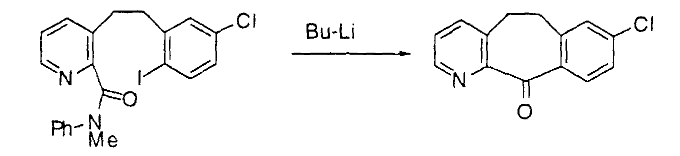

To a solution of 2 g (4.2 mmol) of the product of Step 3 in 20 mL of THF at -78°C was added dropwise 2.52 mL of 2.0 n-BuLi (5.0 mmol) in cyclohexane. The mixture was stirred at -78°C for 10 min. and quenched with 30 mL of saturated NH₄Cl. The quenched solution was extracted with 2 X 50 mL of EtOAc. The combined extract was washed with brine, dried over MgSO₄ and concentrated. The residue was passed through silica gel, eluting with hexane/EtOAc (6:4) to give 1.02 g (78%) of the title compound. ¹H NMR (CDCl₃): δ 8.60 (dd, J = 4.6, 1.5 Hz, 1H), 7.95 (d, J = 8.5 Hz, 1H), 7.55 (dd, J = 7.7, 1.5Hz, 1H), 7.29 (dd, J = 7.7, 4.6 Hz, 1H), 7.24 (dd, J = 8.5, 2.0 Hz, 1H), 7.17 ( d, J = 2.0, 1H), 3.15-3.20 (m, 4H). ¹³C NMR (CDCl₃): b 194.1, 155.3, 149.4, 143.9, 139.6, 138,2, 137.4, 136.4, 133.6, 130.3, 127.9, 126.8, 35.2, 33.1.

### Example 4

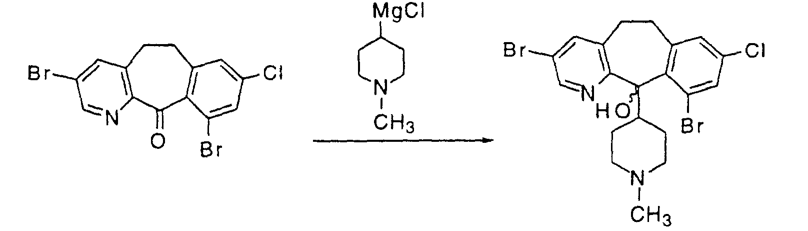

To a solution of 1.88 g (4.68 mmol) of the product of Example 1 in 10 mL THF at -20°C was added dropwise 5.72 mL (5.15 mmol) of 0.9 M of the Grignard. The reaction was stirred at that temperature for 1 hr, quenched into NH₄Cl and extracted with EtOAc. The combined extract was washed with brine, dried over MgSO₄ and concentrated. The residue was chromatographed on silica gel, eluting with EtOAc/hexane to give 1.4 g (60%) product as fluffy solid. Mp. 98-100°C. ¹H NMR (CDCl₃): δ 8.45 (d, J = 2.1 Hz, 1 H), 7.64 (d, J = 2.1 Hz, 1 H), 7.62 (d, J = 2.2 Hz, 1 H), 7.07 (d, J = 2.2 Hz, 1 H), 6.86 (s, 1H), 3.68-3.58 (m, 1 H), 3.48-3.39 (m, 1 H), 3.06-2.8 (m, 4 H), 2.66-2.57 (m, 1 H), 2.23 (s, 3 H), 1.85-1.75 °(m, 2 H), 1.68-1.58 (m, 1 H), 1.40-1.36 (m, 1 H), 0.91-0.85 (m, 1 H). ¹³C NMR (CDCl₃): δ 156.54, 145.04, 141.25, 140.57, 139.09, 135.32, 134.65, 132.52, 130.42, 122.35, 119.64, 80.50, 56.07, 55.70, 45.94, 44.98, 34.27, 30.83, 26.20, 26.09. IR: 3300 (w), 2920 (s), 1570 (w) cm⁻¹.

## Claims

1. A process for preparing a compound of the formula 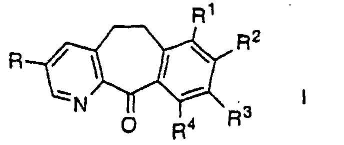 wherein:
R, R¹, R², R³ and R⁴ are independently selected from the group consisting of hydrogen and halo;
comprising:
(a) reacting a compound of formula 1 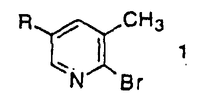
(i) with an amine of the formula NHR⁵R⁶, wherein R⁵ is hydrogen and R⁶ is C₁-C₆ alkyl, aryl or heteroaryl; R⁵ is C₁-C₆ alkyl, aryl or heteroaryl and R⁶ is hydrogen; R⁵ and R⁶ are independently selected from the group consisting of C₁-C₆ alkyl and aryl; or R⁵ and R⁶, together with the nitrogen to which they are attached, form a ring comprising 4 to 6 carbon atoms or comprising 3 to 5 carbon atoms and one hetero moiety selected from the group consisting of -O- and -NR⁹-, wherein R⁹ is H, C₁-C₆ alkyl or phenyl; in the presence of a palladium catalyst and carbon monoxide to obtain an amide of formula 2: 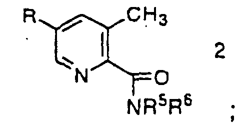 or
(ii) with an alcohol of the formula R¹⁰OH, wherein R¹⁰ is C₁-C₆ lower alkyl or C₃-C₆ cycloalkyl, in the presence of a palladium catalyst and carbon monoxide to obtain the ester of formula 2A 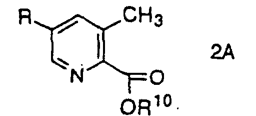 followed by reacting the compound of 2A with an amine of formula NHR⁵R⁶ to obtain the amide of formula 2;
(b) reacting the amide of formula 2 with an iodo-substituted compound of formula 3 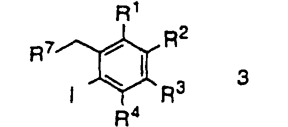 wherein R¹, R², R³ and R⁴ are as defined above and R⁷ is Cl or Br, in the presence of a strong base to obtain a compound of formula 4 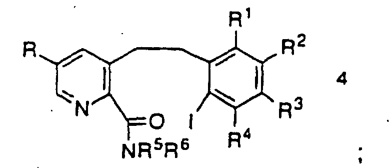
(c) cyclizing a compound of formula 4 with a reagent of the formula R⁸MgL, or when none of R, R¹, R², R³ and R⁴ are bromo, with a reagent of the formula R⁸Li, wherein R⁸ is C₁-C₈ alkyl, aryl or heteroaryl and L is Br or Cl, provided that prior to cyclization, compounds wherein R⁵ or R⁶ is hydrogen are reacted with a suitable N-protecting group.

2. A process of claim 1 for preparing a compound of the formula 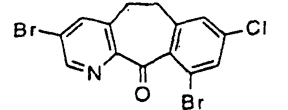 comprising:
(a) reacting a compound of the formula 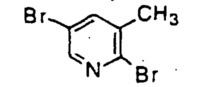
(i) with an amine of the formula NHR⁵R⁶, wherein R⁵ is hydrogen and R⁶ is C₁-C₆ alkyl, aryl or heteroaryl; R⁵ is C₁-C₆ alkyl, aryl or heteroaryl and R⁶ is hydrogen; R⁵ and R⁶ are independently selected from the group consisting of C₁-C₆ alkyl and aryl; or R⁵ and R⁶, together with the nitrogen to which they are attached, form a ring comprising 4 to 6 carbon atoms or comprising 3 to 5 carbon atoms and one hetero moiety selected from the group consisting of -O- and -NR⁹-, wherein R⁹ is H, C₁-C₆ alkyl or phenyl; in the presence of a palladium catalyst and carbon monoxide to obtain an amide of the formula: 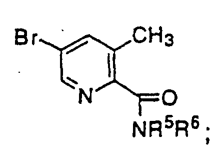 or
(ii) with an alcohol of the formula R¹⁰OH, wherein R¹⁰ is C₁-C₆ lower alkyl or C₃-C₆ cycloalkyl, in the presence of a palladium catalyst and carbon monoxide to obtain the ester of the formula 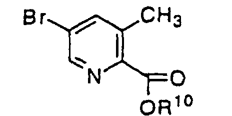 followed by reacting the ester with an amine of formula NHR⁵R⁶ to obtain the amide of step (i);
(b) reacting the amide of step (a) with an iodo-substituted compound of the formula 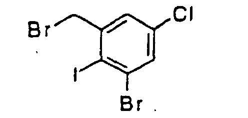 in the presence of a strong base to obtain a compound of the formula 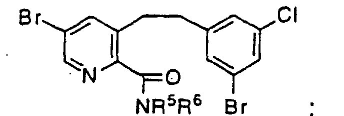
(c) cyclizing a compound of step (b) with a reagent of the formula R⁸MgL, wherein R⁸ is C₁-C₈ alkyl, aryl or heteroaryl and L is Br or Cl, provided that prior to cyclization, compounds wherein R⁵ or R⁶ is hydrogen are reacted with a suitable N-protecting group.

3. A process of claim 1 for preparing a compound of the formula 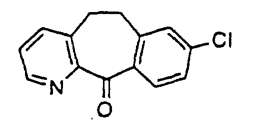 comprising:
(a) reacting a compound of the formula 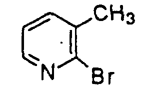
(i) with an amine of the formula NHR⁵R⁶, wherein R⁵ is hydrogen and R⁶ is C₁-C₆ alkyl, aryl or heteroaryl; R⁵ is C₁-C₆ alkyl, aryl or heteroaryl and R⁶ is hydrogen; R⁵ and R⁶ are independently selected from the group consisting of C₁-C₆ alkyl and aryl; or R⁵ and R⁶, together with the nitrogen to which they are attached, form a ring comprising 4 to 6 carbon atoms or comprising 3 to 5 carbon atoms and one hetero moiety selected from the group consisting of -O- and -NR⁹-, wherein R⁹ is H, C₁-C₆ alkyl or phenyl; in the presence of a palladium catalyst and carbon monoxide to obtain an amide of the formula: 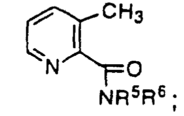 or
(ii) with an alcohol of the formula R¹⁰OH, wherein R¹⁰ is C₁-C₆ lower alkyl or C₃-C₆ cycloalkyl, in the presence of a palladium catalyst and carbon monoxide to obtain the ester of the formula 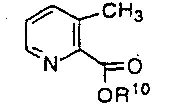 followed by reacting the ester with an amine of formula NHR⁵R⁶ to obtain the amide of step (i);
(b) reacting the amide of step (a) with an iodo-substituted compound of the formula 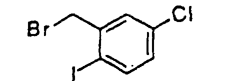 in the presence of a strong base to obtain a compound of the formula 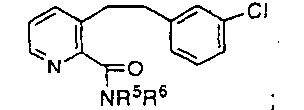
(c) cyclizing a compound of step (b) with a reagent of the formula R⁸MgL or R⁸Li, wherein R⁸ is C₁-C₈ alkyl, aryl or heteroaryl and L is Br or Cl, provided that prior to cyclization, compounds wherein R⁵ or R⁶ is hydrogen are reacted with a suitable N-protecting group.

4. The process of any of claims 1, 2 or 3 wherein R⁵ is phenyl and R⁶ is hydrogen.

5. The process of any of claims 1, 2, 3 or 4 wherein the palladium catalyst is PdX₂/ligand; Pd(PPh₃)₄; (R¹¹)₃P/ Pd₂(dba)₃; or Pd/C, wherein X is OAc or Cl, ligand is P(R¹¹)₃, dipyridyl, 2-aminopyridine, 2-cyano-pyridine, 2-dimethylaminopyridine, 1,10-phenanthroline, 2-methoxy-pyridine or (S)-(-)-nicotine, and wherein Ac is acetyl, R¹¹ is C₁ to C₆ alkyl or aryl, Ph is phenyl, and dba is dibenzylidene acetone.

6. The process of any of claims 1, 2, 3, 4 or 5 wherein R⁸MgL is isopropylmagnesium chloride, 2-mesitylmagnesium bromide, o-tolylmagnesium bromide, 2-methoxy-phenylmagnesium bromide, 2-methoxy-5-methylphenyl-magnesium bromide, 2,5-dimethoxyphenyl-magnesium bromide or N-methyl-piperidylmagnesium bromide.

7. A process of any of claims 1, 3, 4 or 5 wherein R⁸Li is n-butyl-lithium, sec-butyllithium, tert-butyllithium, methyllithium or phenyllithium.

8. A process of claim 7 wherein R⁶ is reacted with a protecting group after step (a) or wherein R⁶ is reacted with a protecting group after step (b).

9. A process for preparing a compound of the formula 5 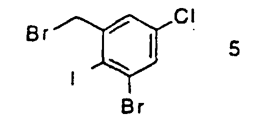 comprising:
i) brominating 2-amino chlorobenzoic acid of formula 6 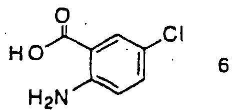 to obtain 2-amino-3-bromo-5-chlorobenzoic acid of formula 7 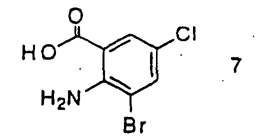
ii) iodonating the compound of formula 7 to obtain 2-iodo-3-bromo-5-chlorobenzoic acid of formula 8 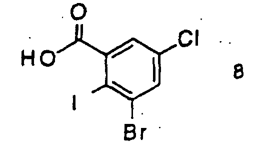
iii) reducing the carboxylic acid of the halo-substituted benzoic acid of formula 8 to obtain the corresponding hydroxy-methyl compound of formula 9 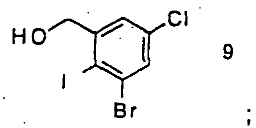 and
iv) brominating the compound of formula 9.

10. A compound which is

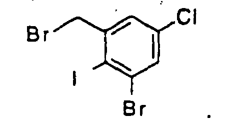

11. A compound of the formula 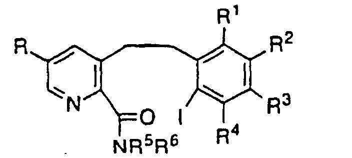 wherein R, R¹, R², R³ and R⁴ are independently selected from the group consisting of hydrogen and halo; and wherein R⁵ is hydrogen and R⁶ is C₁-C₆ alkyl, aryl or heteroaryl; R⁵ is C₁-C₆ alkyl, aryl or heteroaryl and R⁶ is hydrogen; R⁵ and R⁶ are independently selected from the group consisting of C₁-C₆ alkyl and aryl; or R⁵ and R⁶, together with the nitrogen to which they are attached, form a ring comprising 4 to 6 carbon atoms or comprising 3 to 5 carbon atoms and one hetero moiety selected from the group consisting of -O- and -NR⁹-, wherein R⁹ is H, C₁-C₆ alkyl or phenyl.

12. A compound of claim 11 selected from the group consisting of

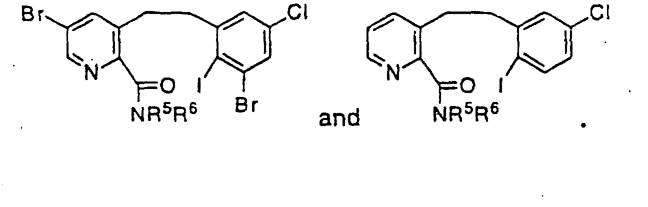

## Patentansprüche

1. Verfahren zum Herstellen einer Verbindung der Formel 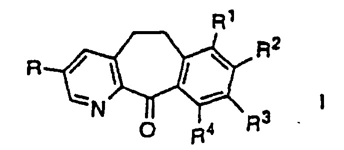 worin:
R, R¹, R², R³ und R⁴ unabhängig aus der aus Wasserstoff und Halogen bestehenden Gruppe ausgewählt sind,
umfassend das:
(a) Umsetzen einer Verbindung der Formel 1 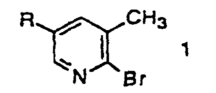
(i) mit einem Amin der Formel NHR⁵R⁶, worin R⁵ Wasserstoff ist und R⁶ C₁-C₆-Alkyl, Aryl oder Heteroaryl ist; R⁵ C₁-C₆-Alkyl, Aryl oder Heteroaryl ist und R⁶ Wasserstoff ist; R⁵ und R⁶ unabhängig aus der aus C₁-C₆-Alkyl und Aryl bestehenden Gruppe ausgewählt sind oder R⁵ und R⁶ zusammen mit dem Stickstoff, an den sie gebunden sind, einen Ring bilden, der 4 bis 6 Kohlenstoffatome umfaßt oder 3 bis 5 Kohlenstoffatome und eine Heterostruktureinheit umfaßt, die aus der aus -O- und -NR⁹-, worin R⁹ H, C₁-C₆-Alkyl oder Phenyl ist, bestehenden Gruppe ausgewählt ist, in Gegenwart eines Palladiumkatalysators und Kohlenmonoxid unter Erhalten eines Amids der Formel 2: 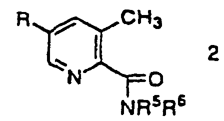 oder
(ii) mit einem Alkohol der Formel R¹⁰OH, worin R¹⁰ C₁-C₆-Niederalkyl oder C₃-C₆-Cycloalkyl ist, in Gegenwart eines Palladiumkatalysators und Kohlenmonoxid unter Erhalten des Esters der Formel 2A 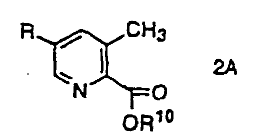 gefolgt vom Umsetzen der Verbindung 2A mit einem Amin der Formel NHR⁵R⁶ unter Erhalten des Amids der Formel 2,
(b) Umsetzen des Amids der Formel 2 mit einer iodsubstituierten Verbindung der Formel 3 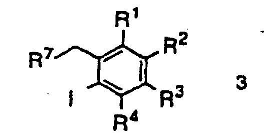 worin R¹, R², R³ und R⁴ wie vorstehend definiert sind und R⁷ Cl oder Br ist, in Gegenwart einer starken Base unter Erhalten einer Verbindung der Formel 4 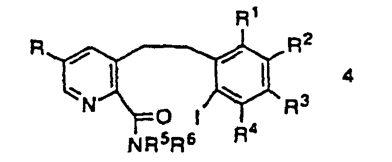
(c) Cyclisieren einer Verbindung der Formel 4 mit einem Reagenz der Formel R⁸MgL, oder wenn keines von R, R¹, R², R³ und R⁴ Brom ist, mit einem Reagenz der Formel R⁸Li, worin R⁸ C₁-C₈-Alkyl, Aryl oder Heteroaryl ist und L Br oder Cl ist, vorausgesetzt, daß vor der Cyclisierung Verbindungen, bei denen R⁵ oder R⁶ Wasserstoff ist, mit einer geeigneten N-Schutzgruppe umgesetzt werden.

2. Verfahren des Anspruchs 1 zum Herstellen einer Verbindung der Formel umfassend das:
(a) Umsetzen einer Verbindung der Formel 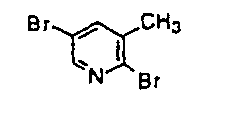
(i) mit einem Amin der Formel NHR⁵R⁶, worin R⁵ Wasserstoff ist und R⁶ C₁-C₆-Alkyl, Aryl oder Heteroaryl ist; R⁵ C₁-C₆-Alkyl, Aryl oder Heteroaryl ist und R⁶ Wasserstoff ist; R⁵ und R⁶ unabhängig aus der aus C₁-C₆-Alkyl und Aryl bestehenden Gruppe ausgewählt sind oder R⁵ und R⁶ zusammen mit dem Stickstoff, an den sie gebunden sind, einen Ring bilden, der 4 bis 6 Kohlenstoffatome umfaßt oder 3 bis 5 Kohlenstoffatome und eine Heterostruktureinheit umfaßt, die aus der aus -O- und -NR⁹-, worin R⁹ H, C₁-C₆-Alkyl oder Phenyl ist, bestehenden Gruppe ausgewählt ist, in Gegenwart eines Palladiumkatalysators und Kohlenmonoxid unter Erhalten eines Amids der Formel 2: 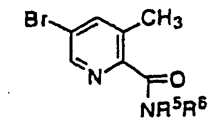 oder
(ii) mit einem Alkohol der Formel R¹⁰OH, worin R¹⁰ C₁-C₆-Niederalkyl oder C₃-C₆-Cycloalkyl ist, in Gegenwart eines Palladiumkatalysators und Kohlenmonoxid unter Erhalten des Esters der Formel 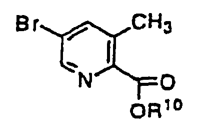 gefolgt vom Umsetzen des Esters mit einem Amin der Formel NHR⁵R⁶ unter Erhalten des Amids aus Schritt (i),
(b) Umsetzen des Amids aus Schritt (a) mit einer iodsubstituierten Verbindung der Formel 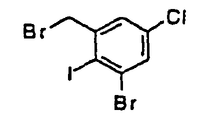 in Gegenwart einer starken Base unter Erhalten einer Verbindung der Formel 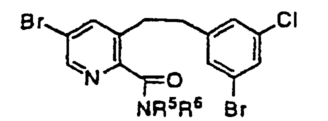
(c) Cyclisieren einer Verbindung aus Schritt (b) mit einem Reagenz der Formel R⁸MgL, worin R⁸ C₁-C₈-Alkyl, Aryl oder Heteroaryl ist und L Br oder Cl ist, vorausgesetzt, daß vor der Cyclisierung Verbindungen, bei denen R⁵ oder R⁶ Wasserstoff ist, mit einer geeigneten N-Schutzgruppe umgesetzt werden.

3. Verfahren des Anspruchs 1 zum Herstellen einer Verbindung der Formel 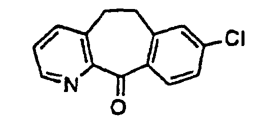 umfassend das:
(a) Umsetzen einer Verbindung der Formel 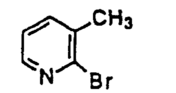
(i) mit einem Amin der Formel NHR⁵R⁶, worin R⁵ Wasserstoff ist und R⁶ C₁-C₆-Alkyl, Aryl oder Heteroaryl ist; R⁵ C₁-C₆-Alkyl, Aryl oder Heteroaryl ist und R⁶ Wasserstoff ist; R⁵ und R⁶ unabhängig aus der aus C₁-C₆-Alkyl und Aryl bestehenden Gruppe ausgewählt sind oder R⁵ und R⁶ zusammen mit dem Stickstoff, an den sie gebunden sind, einen Ring bilden, der 4 bis 6 Kohlenstoffatome umfaßt oder 3 bis 5 Kohlenstoffatome und eine Heterostruktureinheit umfaßt, die aus der aus -O- und -NR⁹-, worin R⁹ H, C₁-C₆-Alkyl oder Phenyl ist, bestehenden Gruppe ausgewählt ist, in Gegenwart eines Palladiumkatalysators und Kohlenmonoxid unter Erhalten eines Amids der Formel: 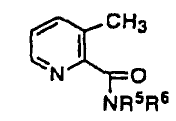 oder
(ii) mit einem Alkohol der Formel R¹⁰OH, worin R¹⁰ C₁-C₆-Niederalkyl oder C₃-C₆-Cycloalkyl ist, in Gegenwart eines Palladiumkatalysators und Kohlenmonoxid unter Erhalten des Esters der Formel 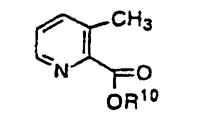 gefolgt vom Umsetzen des Esters mit einem Amin der Formel NHR⁵R⁶ unter Erhalten des Amids aus Schritt (i),
(b) Umsetzen des Amids aus Schritt (a) mit einer iodsubstituierten Verbindung der Formel 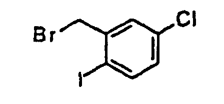 in Gegenwart einer starken Base unter Erhalten einer Verbindung der Formel 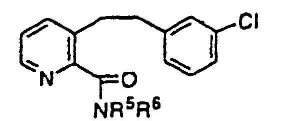
(c) Cyclisieren einer Verbindung aus Schritt (b) mit einem Reagenz der Formel R⁸MgL oder R⁸Li, worin R⁸ C₁-C₈-Alkyl, Aryl oder Heteroaryl ist und L Br oder Cl ist, vorausgesetzt, daß vor der Cyclisierung Verbindungen, bei denen R⁵ oder R⁶ Wasserstoff ist, mit einer geeigneten N-Schutzgruppe umgesetzt werden.

4. Verfahren eines der Ansprüche 1, 2 oder 3, wobei R⁵ Phenyl ist und R⁶ Wasserstoff ist.

5. Verfahren eines der Ansprüche 1, 2, 3 oder 4, wobei der Palladiumkatalysator PdX₂/Ligand, Pd(PPh₃)₄, (R¹¹)₃P/Pd₂(dba)₃ oder Pd/C ist, wobei X OAc oder Cl ist, der Ligand P(R¹¹)₃, Dipyridyl, 2-Aminopyridin, 2-Cyanpyridin, 2-Dimethylaminopyridin, 1,10-Phenanthrolin, 2-Methoxypyridin oder (S)-(-)-Nicotin ist und wobei Ac Acetyl ist, R¹¹ C₁- bis C₆-Alkyl oder Aryl ist, Ph Phenyl ist und dba Dibenzylidenaceton ist.

6. Verfahren eines der Ansprüche 1, 2, 3, 4 oder 5, wobei R⁸MgL Isopropylmagnesiumchlorid, 2-Mesitylmagnesiumbromid, o-Tolylmagnesiumbromid, 2-Methoxyphenylmagnesiumbromid, 2-Methoxy-5-methylphenylmagnesiumbromid, 2,5-Dimethoxyphenylmagnesiumbromid oder N-Methylpiperidylmagnesiumbromid ist.

7. Verfahren eines der Ansprüche 1, 3, 4 oder 5, wobei R⁸Li n-Butyllithium, sec-Butyllithium, tert-Butyllithium, Methyllithium oder Phenyllithium ist.

8. Verfahren des Anspruchs 7, wobei R⁶ nach Schritt (a) mit einer Schutzgruppe umgesetzt wird oder wobei R⁶ nach Schritt (b) mit einer Schutzgruppe umgesetzt wird.

9. Verfahren zum Herstellen einer Verbindung der Formel 5 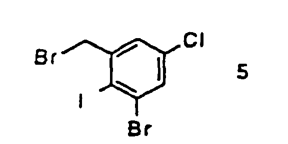 umfassend das:
i) Bromieren von 2-Aminochlorbenzoesäure der Formel 6 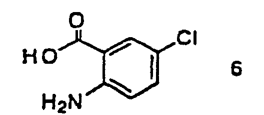 unter Erhalten von 2-Amino-3-brom-5-chlorbenzoesäure der Formel 7 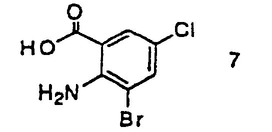
ii) Iodieren der Verbindung der Formel 7 unter Erhalten von 2-Iod-3-brom-5-chlorbenzoesäure der Formel 8 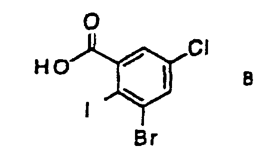
iii) Reduzieren der Carbonsäure der halogensubstituierten Benzoesäure der Formel 8 unter Erhalten der entsprechenden Hydroxymethylverbindung der Formel 9 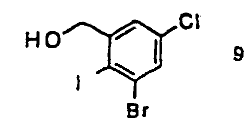 und
iv) Bromieren der Verbindung der Formel 9.

10. Verbindung, die 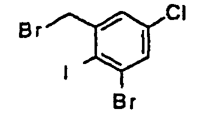 ist.

11. Verbindung der Formel 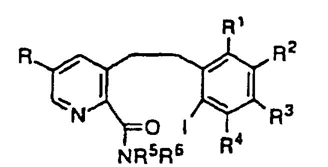 R, R¹, R², R³ und R⁴ unabhängig aus der aus Wasserstoff und Halogen bestehenden Gruppe ausgewählt sind und wobei R⁵ Wasserstoff ist und R⁶ C₁-C₆-Alkyl, Aryl oder Heteroaryl ist; R⁵ C₁-C₆-Alkyl, Aryl oder Heteroaryl ist und R⁶ Wasserstoff ist; R⁵ und R⁶ unabhängig aus der aus C₁-C₆-Alkyl und Aryl bestehenden Gruppe ausgewählt sind oder R⁵ und R⁶ zusammen mit dem Stickstoff, an den sie gebunden sind, einen Ring bilden, der 4 bis 6 Kohlenstoffatome umfaßt oder 3 bis 5 Kohlenstoffatome und eine Heterostruktureinheit umfaßt, die aus der aus -Ound -NR⁹-, worin R⁹ H, C₁-C₆-Alkyl oder Phenyl ist, bestehenden Gruppe ausgewählt ist.

12. Verbindung des Anspruchs 11 ausgewählt aus der aus 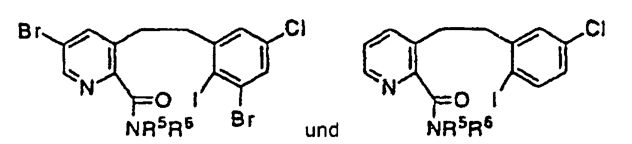 bestehenden Gruppe.

## Revendications

1. Procédé de préparation d'un composé de formule 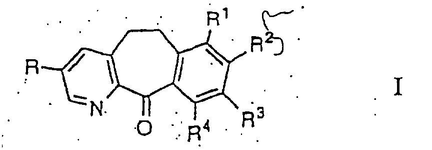 dans laquelle R, R¹, R², R³ et R⁴ sont indépendamment choisis dans l'ensemble formé par les atomes d'hydrogène et d'halogène,
lequel procédé comporte :
a) le fait de faire réagir un composé de formule 1 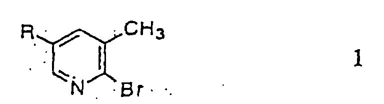
i) avec une amine de formule NHR⁵R⁶, dans laquelle
- R⁵ représente un atome d'hydrogène et R⁶ représente un groupe alkyle en C₁₋₆, aryle ou hétéroaryle,
- R⁵ représente un groupe alkyle en C₁₋₆, aryle ou hétéroaryle et R⁶ représente un atome d'hydrogène,
- R⁵ et R⁶ sont indépendamment choisis dans l'ensemble formé par les groupes alkyle en C₁₋₆ et aryle,
- ou R⁵ et R⁶ forment, conjointement avec l'atome d'azote auquel ils sont liés, un cycle comportant de 4 à 6 atomes de carbone ou comportant de 3 à 5 atomes de carbone et un hétéro-chaînon choisi parmi -O- et -NR⁹- où R⁹ représente un atome d'hydrogène ou un groupe alkyle en C₁₋₆ ou phényle,
en présence d'un catalyseur au palladium et de monoxyde de carbone, pour obtenir un amide de formule 2 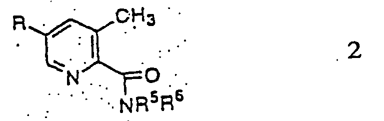
ii) ou avec un alcool de formule R¹⁰OH, dans laquelle R¹⁰ représente un groupe alkyle inférieur en C₁₋₆ ou cycloalkyle en C₃₋₆, en présence d'un catalyseur au palladium et de monoxyde de carbone, pour obtenir un ester de formule 2A 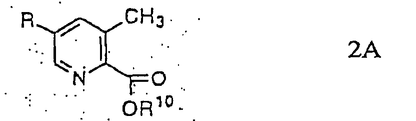 et le fait de faire ensuite réagir ce composé de formule 2A avec une amine de formule NHR⁵R⁶, pour obtenir un amide de formule 2 ;
b) le fait de faire réagir l'amide de formule 2 avec un composé iodo-substitué de formule 3 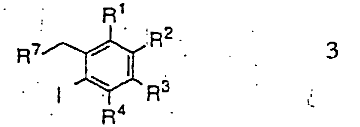 dans laquelle R¹, R², R³ et R⁴ ont les significations indiquées plus haut et R⁷ représente un atome de chlore ou de brome, en présence d'une base forte, pour obtenir un composé de formule 4 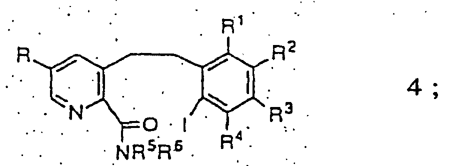
c) et le fait de cycliser le composé de formule 4, à l'aide d'un réactif de formule R⁸MgL ou, si aucun des symboles R, R¹, R², R³ et R⁴ ne représente un atome de brome, à l'aide d'un réactif de formule R⁸Li, dans lesquelles formules R⁸ représente un groupe alkyle en C₁₋₈, aryle ou hétéroaryle et L représente un atome de brome ou de chlore, sous réserve qu'avant cette cyclisation, on fasse réagir avec un groupe amino-protecteur approprié les composés dans lesquels R⁵ ou R⁶ représente un atome d'hydrogène.

2. Procédé, conforme à la revendication 1, de préparation d'un composé de formule 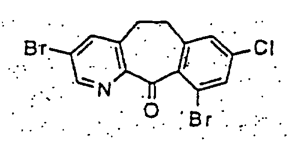 lequel procédé comporte :
a) le fait de faire réagir un composé de formule 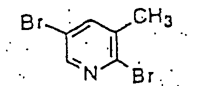
i) avec une amine de formule NHR⁵R⁶, dans laquelle
- R⁵ représente un atome d'hydrogène et R⁶ représente un groupe alkyle en C₁₋₆, aryle ou hétéroaryle,
- R⁵ représente un groupe alkyle en C₁₋₆, aryle ou hétéroaryle et R⁶ représente un atome d'hydrogène,
- R⁵ et R⁶ sont indépendamment choisis dans l'ensemble formé par les groupes alkyle en C₁₋₆ et aryle,
- ou R⁵ et R⁶ forment, conjointement avec l'atome d'azote auquel ils sont liés, un cycle comportant de 4 à 6 atomes de carbone ou comportant de 3 à 5 atomes de carbone et un hétéro-chaînon choisi parmi -O- et -NR⁹- où R⁹ représente un atome d'hydrogène ou un groupe alkyle en C₁₋₆ ou phényle,
en présence d'un catalyseur au palladium et de monoxyde de carbone, pour obtenir un amide de formule 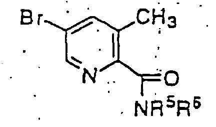
ii) ou avec un alcool de formule R¹⁰OH, dans laquelle R¹⁰ représente un groupe alkyle inférieur en C₁₋₆ ou cycloalkyle en C₃₋₆,
en présence d'un catalyseur au palladium et de monoxyde de carbone, pour obtenir un ester de formule 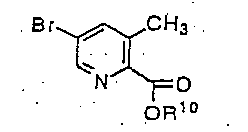 et le fait de faire ensuite réagir cet ester avec une amine de formule NHR⁵R⁶, pour obtenir l'amide indiqué dans l'étape (i) ;
b) le fait de faire réagir l'amide obtenu dans l'étape (a) avec un composé iodo-substitué de formule 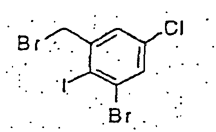 en présence d'une base forte, pour obtenir un composé de formule 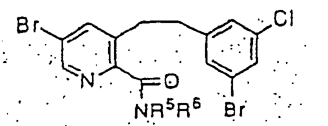
c) et le fait de cycliser le composé obtenu dans l'étape (b), à l'aide d'un réactif de formule R⁸MgL dans laquelle R⁸ représente un groupe alkyle en C₁₋₈, aryle ou hétéroaryle et L représente un atome de brome ou de chlore, sous réserve qu'avant cette cyclisation, on fasse réagir avec un groupe amino-protecteur approprié les composés dans lesquels R⁵ ou R⁶ représente un atome, d'hydrogène.

3. Procédé, conforme à la revendication 1, de préparation d'un composé de formule 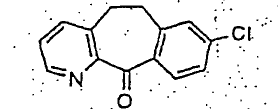 lequel procédé comporte :
a) le fait de faire réagir un composé de formule 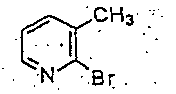
i) avec une amine de formule NHR⁵R⁶, dans laquelle
- R⁵ représente un atome d'hydrogène et R⁶ représente un groupe alkyle en C₁₋₆, aryle ou hétéroaryle,
- R⁵ représente un groupe alkyle en C₁₋₆, aryle ou hétéroaryle et R⁶ représente un atome d'hydrogène,
- R⁵ et R⁶ sont indépendamment choisis dans l'ensemble formé par les groupes alkyle en C₁₋₆ et aryle,
- ou R⁵ et R⁶ forment, conjointement avec l'atome d'azote auquel ils sont liés, un cycle comportant de 4 à 6 atomes de carbone ou comportant de 3 à 5 atomes de carbone et un hétéro-chaînon choisi parmi -O- et -NR⁹- où R⁹ représente un atome d'hydrogène ou un groupe alkyle en C₁₋₆ ou phényle,
en présence d'un catalyseur au palladium et de monoxyde de carbone, pour obtenir un amide de formule. 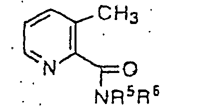
ii) ou avec un alcool de formule R¹⁰OH; dans laquelle R¹⁰ représente un groupe alkyle inférieur en C₁₋₆ ou cycloalkyle en C₃₋₆, en présence d'un catalyseur au palladium et de monoxyde de carbone, pour obtenir un ester de formule 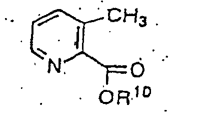 et le fait de faire ensuite réagir cet ester avec une amine de formule NHR⁵R⁶, pour obtenir l'amide indiqué dans l'étape (i) ;
b) le fait de faire réagir l'amide obtenu dans l'étape (a) avec un composé iodo-substitué de formule 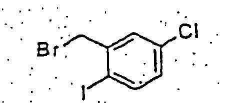 en présence d'une base forte, pour obtenir un composé de formule 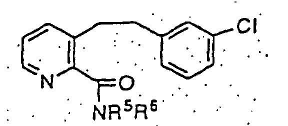
c) et le fait de cycliser le composé obtenu dans l'étape (b), à l'aide d'un réactif de formule R⁸MgL ou R⁸Li, dans lesquelles formules R⁸ représente un groupe alkyle en C₁₋₈, aryle ou hétéroaryle et L représente un atome de brome ou de chlore, sous réserve qu'avant cette cyclisation, on fasse réagir avec un groupe amino-protecteur approprié les composés dans lesquels R⁵ ou R⁶ représente un atome d'hydrogène.

4. Procédé conforme à l'une des revendications 1, 2 et 3, dans lequel R⁵ représente un groupe phényle et R⁶ représente un atome d'hydrogène.

5. Procédé conforme à l'une des revendications 1, 2, 3 et 4, dans lequel le catalyseur au palladium est du Pd/C ou un composé de formule PdX₂/ligand, Pd(PPh₃)₄ ou (R¹¹)₃P/Pd₂(dba)₃, où X représente un groupe OAc ou un atome de chlore, "ligand" représente P(R¹¹)₃, dipyridyle, 2-aminopyridine, 2-cyanopyridine, 2-diméthylaminopyridine, 1,10-phénanthroline, 2-méthoxypyridine ou (S)-(-)-nicotine, Ac représente un groupe acétyle, R¹¹ représente un groupe alkyle en C₁₋₆ ou aryle, Ph représente un groupe phényle et "dba" représente la dibenzylidène-acétone.

6. Procédé conforme à l'une des revendications 1, 2, 3, 4 et 5, dans lequel R⁸MgL est du chlorure d'isopropyl-magnésium, du bromure de 2-mésityl-magnésium, du bromure d'o-tolyl-magnésium, du bromure de 2-méthoxyphényl-magnésium, du bromure de 2-méthoxy-5-méthylphényl-magnésium, du bromure de 2,5-diméthoxyphényl-magnésium, ou du bromure de N-méthyl-pipéridyl-magnésium.

7. Procédé conforme à l'une des revendications 1, 3, 4 et 5, dans lequel R⁸Li est du n-butyl-lithium, du sec-butyl-lithium, du tert-butyl-lithium, du méthyl-lithium ou du phényl-lithium.

8. Procédé conforme à la revendication 7, dans lequel on fait réagir R⁶ avec un groupe protecteur après l'étape (a), ou bien l'on fait réagir R⁶ avec un groupe protecteur après l'étape (b).

9. Procédé de préparation d'un composé de formule 5 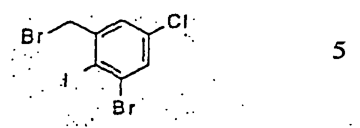 lequel procédé comporte :
i) le fait de bromer de l'acide 2-amino-chlorobenzoïque de formule 6 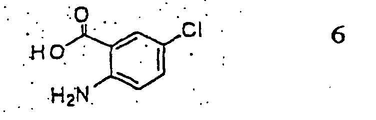 pour obtenir de l'acide 2-amino-3-bromo-5-chlorobenzoïque de formule 7 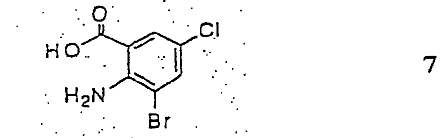
ii) le fait de ioder du composé de formule 7, pour obtenir de l'acide 2-iodo-3-bromo-5-chlorobenzoïque de formule 8 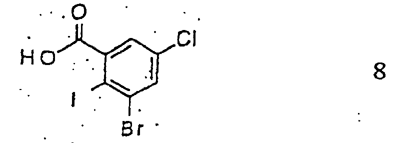
iii) le fait de réduire la fonction acide carboxylique de l'acide halogénobenzoïque de formule 8, pour obtenir le composé hydroxyméthylé correspondant de formule 9 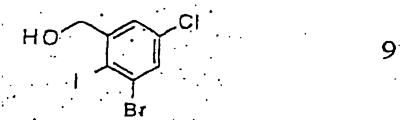
iv) et le fait de bromer le composé de formule 9

10. Composé représenté par la formule :

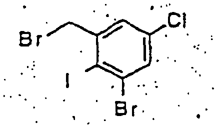

11. Composé de formule 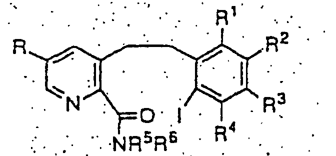 dans laquelle R, R¹, R², R³ et R⁴ sont indépendamment choisis dans l'ensemble formé par les atomes d'hydrogène et d'halogène,
et dans laquelle
- R⁵ représente un atome d'hydrogène et R⁶ représente un groupe alkyle en C₁₋₆, aryle ou hétéroaryle,
- R⁵ représente un groupe alkyle en C₁₋₆, aryle ou hétéroaryle et R⁶ représente un atome d'hydrogène,
- R⁵ et R⁶ sont indépendamment choisis dans l'ensemble formé par les groupes alkyle en C₁₋₆ et aryle,
- ou R⁵ et R⁶ forment, conjointement avec l'atome d'azote auquel ils sont liés, un cycle comportant de 4 à 6 atomes de carbone ou comportant de 3 à 5 atomes de carbone et un hétéro-chaînon choisi parmi -O- et -NR⁹- où R⁹ représente un atome d'hydrogène ou un groupe alkyle en C₁₋₆ ou phényle.

12. Composé conforme à la revendication 11, choisi dans l'ensemble formé par les composés de formules 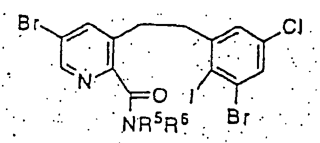 et
